# EUROPÄISCHE PATENTSCHRIFT

(11) **EP 2 379 481 B1**
(45) Veröffentlichungstag und Bekanntmachung des Hinweises auf die Patenterteilung: **26.08.2015**
(21) Anmeldenummer: 09795970.4
(22) Anmeldetag: 15.12.2009
(51) Int. Cl.: C07C 67/08, C07C 69/00, C07C 69/80

(54) **VERFAHREN ZUR HERSTELLUNG VON CARBONSÄUREESTERN**
METHOD FOR PRODUCING CARBOXYLIC ACID ESTERS
PROCÉDÉ DE FABRICATION D'ESTERS D'ACIDES CARBOXYLIQUES

(30) Priorität: 16.12.2008 EP 08171796
(43) Veröffentlichungstag der Anmeldung: 26.10.2011
(73) Patentinhaber: BASF SE, 67056 Ludwigshafen (DE)
(72) Erfinder: DISTELDORF, Walter, 67157 Wachenheim (DE); PETERS, Jarren, 68163 Mannheim (DE); SCHÄFER, Thomas, 68165 Mannheim (DE); FRIESE, Katrin, 68199 Mannheim (DE); ÜBLER, Christoph, 67308 Lautersheim (DE)
(86) Internationale Anmeldenummer: PCT/EP2009/067177
(87) Internationale Veröffentlichungsnummer: WO 2010/076192

(56) Entgegenhaltungen:
- EP-A2- 1 186 593
- WO-A1-00/78702
- US-A1- 2004 192 957

## Beschreibung

Die Erfindung betrifft ein Verfahren zur Herstellung von Carbonsäureestern durch Umsetzung einer Carbonsäure oder eines Carbonsäureanhydrids oder eines Gemisches davon mit einem Alkohol.

Ester der Phthalsäure, Adipinsäure, Sebacinsäure oder Maleinsäure finden weite Anwendung in Lackharzen, als Bestandteile von Anstrichmitteln und insbesondere als Weichmacher für Kunststoffe.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Unabhängig von der Art der Katalyse entsteht immer ein temperaturabhängiges Gleichgewicht zwischen den Einsatzstoffen (Carbonsäure und Alkohol) und den Produkten (Ester und Wasser).

Die Umsetzung von inneren Carbonsäureanhydriden mit Alkoholen verläuft in zwei Schritten: Die Alkoholyse des Anhydrids zum Monoester verläuft in der Regel rasch und vollständig. Die weitere Umsetzung des Monoesters zum Diester unter Bildung von Reaktionswasser ist reversibel und verläuft langsam. Dieser zweite Schritt ist der geschwindigkeitsbestimmende Schritt der Reaktion.

Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel.

Die EP-A 1 186 593 beschreibt ein Verfahren zur Herstellung von Carbonsäureestern durch Reaktion von Di- oder Polycarbonsäuren oder deren Anhydride mit Alkoholen, wobei das Reaktionswasser durch azeotrope Destillation mit dem Alkohol entfernt wird. Die durch die azeotrope Destillation aus der Reaktion entfernte Flüssigkeitsmenge wird vollständig oder teilweise mit dem Alkohol wieder ergänzt.

Dient der eingesetzte Alkohol als Schleppmittel, geht man üblicherweise so vor, dass man den Brüden aus dem Reaktor zumindest teilweise kondensiert, das Kondensat in eine wässrige Phase und eine im Wesentlichen aus dem zur Veresterung eingesetzten Alkohol bestehende organische Phase trennt und die organische Phase zumindest teilweise in den Reaktor zurückführt. Allerdings entstehen bei der Veresterungsreaktion neben dem gewünschten Ester verschiedene Nebenprodukte. Vor allem die niedriger als der Alkohol siedenden Nebenprodukte werden mit der organischen Phase in den Reaktor zurückgeführt und können sich, insbesondere bei kontinuierlicher Verfahrensführung, im Reaktionssystem anreichern.

Es wurde gefunden, dass die niedriger als der Alkohol siedenden Nebenprodukte hauptsächlich aus Olefinen bestehen, die durch Wasserabspaltung aus dem eingesetzten Alkohol gebildet werden. Höhere Konzentrationen der Olefine können den eingesetzten Veresterungskatalysator schädigen und/oder die Qualität des hergestellten Esterprodukts beeinträchtigen, insbesondere zu unerwünschten Verfärbungen führen. Um eine Anreicherung der niedriger als der Alkohol siedenden Nebenprodukte im Reaktionssystem zu vermeiden, ist man darauf angewiesen, die organische Phase nicht vollständig in den Reaktor zurückzuführen, sondern einen Teilstrom auszuschleusen. Mit dem Ausschleusstrom geht aber ein beträchtlicher Anteil des eingesetzten Alkohols für die Veresterungsreaktion verloren.

Der Erfindung liegt daher die Aufgabe zugrunde, die Alkoholverluste über den Ausschleusstrom zu minimieren.

Gegenstand der vorliegenden Erfindung ist daher ein Verfahren zur Herstellung von Carbonsäureestern durch Umsetzung einer Carbonsäure oder eines Carbonsäureanhydrids oder eines Gemisches davon mit einem Alkohol in Gegenwart eines Veresterungskatalysators, der unter Alkoholaten, Carboxylaten und Chelatverbindungen von Titan, Zirkonium, Zinn, Aluminium und Zink ausgewählt ist, in einem aus einem oder mehreren Reaktoren bestehenden Reaktionssystem, wobei man Reaktionswasser als Alkohol-Wasser-Azeotrop mit dem Brüden abdestilliert, den Brüden zumindest teilweise kondensiert, das Kondensat in eine wässrige Phase und eine organische Phase trennt und die organische Phase zumindest teilweise in das Reaktionssystem zurückführt, dadurch gekennzeichnet, dass man aus der zurückzuführenden organischen Phase niedriger als der Alkohol siedende Komponenten zumindest teilweise entfernt, wobei die niedriger als der Alkohol siedenden Komponenten Olefine umfassen, die sich durch Wasserabspaltung vom eingesetzten Alkohol ableiten.

Unter "Reaktionssystem" wird ein Reaktor oder eine Anordnung von mehreren Reaktoren verstanden. Mehrere Reaktoren sind vorzugsweise hintereinander geschaltet. "Rückführung in das Reaktionssystem" bedeutet, dass eine organische Phase in wenigstens einen beliebigen Reaktor des Reaktionssystems geleitet wird. Das erfindungsgemäße Verfahren kann diskontinuierlich oder kontinuierlich durchgeführt werden, wird aber vorzugsweise kontinuierlich durchgeführt. Die Leichtsieder umfassen bzw. bestehen im Wesentlichen aus Olefinen (in derRegel Olefin-Isomerengemischen), die sich durch Wasserabspaltung vom eingesetzten Alkohol ableiten.

Bei den Reaktoren kann es sich um beliebige Reaktoren handeln, die zur Durchführung von chemischen Umsetzungen in flüssiger Phase geeignet sind.

Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Es können aber auch Kombinationen aus aufeinander folgenden rückvermischten Reaktoren und nicht rückvermischten Reaktoren verwendet werden.

Gegebenenfalls können auch mehrere Reaktoren in einer mehrstufigen Apparatur zusammengefasst werden. Solche Reaktoren sind zum Beispiel Schlaufenreaktoren mit eingebauten Siebböden, kaskadierte Behälter, Rohrreaktoren mit Zwischeneinspeisung oder Rührkolonnen.

In einer weiteren Verfahrensvariante kann die Umsetzung in einer Reaktivdestillationskolonne durchgeführt werden. Diese Kolonnen zeichnen sich durch eine hohe Verweilzeit der Reaktionslösung in der jeweiligen Stufe aus. So können vorteilhaft z.B. Kolonnen verwendet werden, die einen hohen Flüssigkeits-"hold-up" haben, wie z.B. bei hochaufgestauten Böden einer Bodenkolonne.

Vorzugsweise werden Rührkesselreaktoren verwendet: Die Rührkesselreaktoren bestehen meist aus metallischen Werkstoffen, wobei Edelstahl bevorzugt ist. Der Reaktionsansatz wird vorzugsweise mit Hilfe eines Rührers oder einer Umlaufpumpe intensiv vermischt.

Wenngleich das erfindungsgemäße Verfahren auch mit nur einem Reaktor betrieben werden kann, ist es doch für eine möglichst vollständige Umsetzung zweckmäßig, mehrere Reaktoren, z. B. Rührkessel, miteinander in Form einer Reaktorkaskade zu verbinden. Die einzelnen Reaktoren werden vom Reaktionsgemisch nacheinander durchlaufen, wobei der Ablauf des ersten Reaktors dem zweiten Reaktor, der Ablauf des zweiten Reaktors dem dritten Reaktor usw. zugeführt wird. Die Reaktorkaskade kann z. B. 2 bis 10 Stufen umfassen, wobei 4 bis 6 Stufen bevorzugt sind.

Während der Reaktion wird ein Alkohol-Wasser-Gemisch als Azeotrop aus der Reaktionsmischung abdestilliert. Während der Reaktion wird außerdem Alkohol in den Reaktor bzw. die einzelnen Reaktoren des Reaktionssystems nachgespeist. Zweckmäßigerweise speist man einen vorgegebenen Mengenstrom Alkohol in den jeweiligen Reaktor ein. Eine Anpassung des Mengenstroms kann in Abhängigkeit von der periodisch gemessenen Säurezahl des Reaktionsgemisches im jeweiligen Reaktor erfolgen.

Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Der Brüden aus den einzelnen Rührkesseln einer Kaskade kann vereinigt werden und gemeinsam der erfindungsgemäßen Leichtsiederabtrennung unterzogen werden. Es hat sich nämlich gezeigt, dass sogar nur ein Kondensator und eine Vorrichtung zur Leichtsiederabtrennung ausreichen können, um den aus mehreren Kesseln einer Kaskade entstammenden Brüden wirksam zu behandeln.

Gegebenenfalls kann man jeweils mehrere Kessel der Kaskade zu einer Untereinheit zusammenfassen, wobei dann jeweils die Untereinheiten mit einem Kondensator und einer Vorrichtung zur Leichtsiederabtrennung gekoppelt sind. Es besteht daneben weiterhin die Möglichkeit, jeden Kessel der Kaskade mit einem Kondensator zu koppeln.

Die erfindungsgemäß behandelte, zurückzuführende organische Phase kann in einen beliebigen Reaktor einer Kaskade geleitet oder auf mehrere Reaktoren der Kaskade aufgeteilt werden. Es ist jedoch bevorzugt, die erfindungsgemäß behandelte, zurückzuführende organische Phase nicht in den letzten Reaktor der Kaskade zu leiten. Vorzugsweise leitet man die erfindungsgemäß behandelte, zurückzuführende organische Phase ausschließlich oder überwiegend in den ersten Reaktor der Kaskade.

Für die Rückführung der organischen Phase in das Reaktionssystem gibt es verschiedene Möglichkeiten. Eine Möglichkeit ist, die organische Phase, gegebenenfalls nach Erwärmen, in das flüssige Reaktionsgemisch zu pumpen.

Zur thermischen Optimierung des Verfahrens führt man die organische Phase aber vorzugsweise über eine Kolonne (so genannte Rückalkohol-Kolonne) in das Reaktionssystem zurück, in der man der rückgeführten organischen Phase zumindest einen Teil des Brüden entgegenführt. Zweckmäßigerweise führt man die organische Phase am Kopf oder im oberen Bereich in die Rückalkohol-Kolonne ein. Das ablaufende Kondensat der Rückalkohol-Kolonne gelangt in das Reaktionssystem zurück, bei Verwendung einer Reaktorkaskade vorzugsweise in den ersten Reaktor. Die Rückführung der organischen Phase über die Rückalkohol-Kolonne weist den Vorteil auf, dass die rückgeführte organische Phase vorerwärmt und von Wasserspuren befreit wird, die nach der Phasentrennung in der organischen Phase verblieben sind bzw. gemäß ihrer thermodynamischen Löslichkeit in der organischen Phase gelöst sind. Bei der Rückalkohol-Kolonne kann es sich beispielsweise um eine Bodenkolonne, Packungskolonne oder Füllkörperkolonne handeln. Eine geringe Trennstufenzahl ist im Allgemeinen ausreichend. Geeignet ist z. B. eine Kolonne mit 2 bis 10 theoretischen Trennstufen.

Bei Verwendung einer Reaktorkaskade verlässt der Brüden vorzugsweise zumindest den ersten Reaktor über die Rückalkohol-Kolonne. Ein oder mehrere oder alle weitere Reaktoren können ebenfalls einen Brüdenabzug zur Rückalkohol-Kolonne aufweisen.

Erfindungsgemäß werden aus der zurückzuführenden organischen Phase niedriger als der Alkohol siedende Komponenten zumindest teilweise entfernt. Hierbei nutzt man die Siedepunktsunterschiede zwischen Olefin/Alkohol bzw. Olefin-Wasser-Azeotrop/ Alkohol-Wasser-Azeotrop aus. Die Siedepunktsreihenfolge ist nachstehend am Beispiel von 1-Nonen/1-Nonanol bzw. deren Azeotrope mit Wasser veranschaulicht:

| | Siedepunkt [°C] |
|---|---|
| 1-Nonen/Wasser Minimum-Hetero-Azeotrop | 94,327 |
| 1-Nonanol/Wasser Minimum-Hetero-Azeotrop | 99, 719 |
| 1-Nonen | 146,903 |
| 1-Nonanol | 213,396 |

In einer Ausführungsform des Verfahrens kondensiert man den Alkohol-Wasser-Azeotrop enthaltenden Brüden unvollständig, wobei sich leichter als der Alkohol siedende Komponenten im nicht kondensierten Brüden anreichern und mit dem nicht kondensierten Brüden ausgeschleust werden können. Das Kondensat wird in eine wässrige Phase und eine organische Phase, die im Wesentlichen aus Alkohol besteht, getrennt und die organische Phase zumindest teilweise in das Reaktionssystem zurückgeführt.

Eine unvollständige Kondensation des Brüden kann durch geeignete Wahl der Temperatur im Kondensator, z. B. durch Wahl der Temperatur und/oder Durchflussmenge des Kühlmediums, erreicht werden. Zur unvollständige Kondensation des Brüden kann man diesen auch, z. B. als Sumpf- oder Seitenzulauf, in eine Kolonne einleiten. Der nicht kondensierte Brüden kann in einem Nachkühler kondensiert werden und z. B. einer thermischen Verwertung zugeführt werden.

In dieser Ausführungsform erfolgt die Trennung von Alkohol und Leichtsiedern in Gegenwart von Wasser; die Trennung beruht auf den unterschiedlichen Siedepunkten des Alkohol-Wasser-Azeotrops und des Olefin-Wasser-Azeotrops. Wie die vorstehende Tabelle zeigt, ist die Siedepunktsdifferenz zwischen den Azeotropen nicht ausgeprägt, so dass mit einer geringen Trennstufenzahl nur eine unvollvollständige Trennung möglich ist. Außerdem kann es zur Ausbildung komplexer Gemische kommen, die neben dem Alkohol-Wasser-Azeotrop und dem Olefin-Wasser-Azeotrop noch Alkohol, Olefin usw. enthalten. Da der Siedepunkt des Alkohol-Wasser-Azeotrops in der Regel niedriger ist als der Siedepunkt des Olefins, wird in dieser Ausführungsform nur eine unvollkommene Abtrennung der Leichtsieder erzielt. Der nicht kondensierte Brüden enthält daher noch einen großen Anteil an Alkohol, der für die Veresterungsreaktion verloren ist.

In einer anderen, bevorzugten Ausführungsform des Verfahrens geht man daher so vor, dass man den Alkohol-Wasser-Azeotrop enthaltenden Brüden zumindest teilweise kondensiert, insbesondere im Wesentlichen vollständig kondensiert, und das Kondensat in eine wässrige Phase und eine organische Phase trennt. Zumindest ein Teil der organischen Phase wird behandelt, indem man niedriger als der Alkohol siedende Komponenten abdampft und/oder abdestilliert, und die so behandelte organische Phase zumindest teilweise in das Reaktionssystem zurückgeführt. Die abgedampften bzw. abdestillierten Leichtsieder können in einem Nachkühler kondensiert werden und z. B. einer thermischen Verwertung zugeführt werden.

In dieser Ausführungsform erfolgt die Trennung von Alkohol und Leichtsiedern in weitgehender Abwesenheit von Wasser. Da der Alkohol und das Olefin in der Regel eine große Siedepunktsdifferenz aufweisen (vgl. die vorstehende Tabelle), ist eine einfache Destillation oder eine Destillation mit geringer Trennstufenzahl meist ausreichend, um eine weitgehende Abtrennung der Leichtsieder zu erzielen.

Um eine Anreicherung von Leichtsiedern im Reaktionssystem zu vermeiden, hat es sich als ausreichend erwiesen, lediglich einen Teil der organischen Phase vor deren Rückführung in das Reaktionssystem zu behandeln. In einer geeigneten Ausführungsform führt man daher einen Teil der organischen Phase unverändert in das Reaktionssystem zurück und behandelt einen anderen Teil der organischen Phase, indem man niedriger als der Alkohol siedende Komponenten abdampft und/oder abdestilliert, und führt die so behandelte organische Phase zumindest teilweise in das Reaktionssystem zurück. Vorzugsweise behandelt man wenigstens 20 % der gesamten organischen Phase, insbesondere 25 bis 60 %, z. B. 30 bis 40 % der gesamten organischen Phase, die bei der Phasentrennung anfällt.

Das Abdampfen bzw. Abdestillieren der niedriger als der Alkohol siedenden Komponenten kann in beliebigen hierzu geeigneten Vorrichtungen erfolgen, z. B. einer Destillationskolonne oder einem Verdampfer beliebiger Bauart, z. B. Rührwerksverdampfer, Schrägrohr-Verdampfer, Senkrechtrohr-Verdampfer mit natürlichem oder Zwangsumlauf, Kletterverdampfer, Fallfilmverdampfer, Horizontalrohr-Verdampfer, Robert-Verdampfer, Herbert-Verdampfer, Tauchrohr-Verdampfer, Spiral-Verdampfer, Plattenverdampfer, Sambay-Verdampfer, oder ähnlichen Vorrichtungen. Das Sumpfprodukt der Kolonne bzw. des Verdampfers wird zumindest teilweise in das Reaktionssystem zurückgeführt.

Als Kolonnen sind z. B. Bodenkolonne, Packungskolonnen oder Füllkörperkolonnen geeignet. Zweckmäßigerweise gibt man einen Teil des Kopfkondensats, gegebenenfalls nach Phasentrennung und Abtrennung der mitgeführten wässrigen Phase, als Rücklauf zurück in die Kolonne. Der andere Teil des Kopfkondensats wird aus dem Verfahren ausgeschleust.

In vielen Fällen ist eine Entspannungsverdampfung geeignet. Hierzu entspannt man zumindest einen Teil der organischen Phase in einen Entspannungsbehälter, wobei niedriger als der Alkohol siedende Komponenten zumindest teilweise verdampfen, und führt die unverdampfte flüssige Phase zumindest teilweise in das Reaktionssystem zurück. Die Druckdifferenz bei der Entspannung beträgt z. B. wenigstens 500 mbar auf einen Enddruck von weniger als 500 mbar, vorzugsweise weniger als 200 mbar. Gegebenenfalls kann man die organische Phase vor der Entspannung durch indirekten Wärmetausch erwärmen. Geeignete Wärmetauscher sind z. B. Rohrbündel-Wärmeaustauscher, Doppelrohr-Wärmeaustauscher, Plattenwärmetauscher, Spiral-Wärmeaustauscher, Rippenrohr-Wärmetauscher und dergleichen. Die Temperatur der organischen Phase vor der Entspannung bemisst sich nach den Siedepunkten des Alkohols bzw. des Olefins und nach der Druckdifferenz bei der Entspannung. Vorzugsweise ist die Temperatur ausreichend, die Leichtsieder bei der Entspannung im wesentlichen vollständig zu verdampfen.

Das erfindungsgemäße Verfahren ist prinzipiell auf alle Veresterungen anwendbar, bei denen das Reaktionswasser als Azeotrop mit einem Alkohol destillativ abgetrennt wird.

Im erfindungsgemäßen Verfahren werden als Säurekomponente Carbonsäuren oder Carbonsäureanhydride eingesetzt. Bei mehrbasigen Carbonsäuren können auch teilweise anhydridisierte Verbindungen eingesetzt werden. Ebenso ist es möglich, Gemische aus Carbonsäuren und Anhydriden zu verwenden.

Die Säuren können aliphatisch, einschließlich carbocyclisch, heterocyclisch, gesättigt oder ungesättigt, sowie aromatisch, einschließlich heteroaromatisch, sein.

Zu den geeigneten Carbonsäuren zählen aliphatische Monocarbonsäuren mit wenigsten 5 Kohlenstoffatomen, insbesondere 5 bis 20 Kohlenstoffatomen, wie n-Pentansäure, 2-Methylbuttersäure, 3-Methylbuttersäure, 2-Methylpentansäure, 2-Ethylbuttersäure, n-Heptansäure, 2-Methylhexansäure, Isoheptansäuren, Cyclohexancarbonsäure, n-Octansäure, 2-Ethylhexansäure, Isooctansäuren, n-Nonansäure, 2-Methyloctansäure, Isononansäuren, n-Decansäure, Isodecansäuren, 2-Methylundecansäure, Isoundecansäure, Tricyclodecancarbonsäure und Isotridecansäure.

Weiter eignen sich aliphatische C₄-C₁₀-Dicarbonsäuren bzw. deren Anhydride, wie z. B. Maleinsäure, Fumarsäure, Maleinsäureanhydrid, Bernsteinsäure, Bernsteinsäureanhydrid, Adipinsäure, Korksäure, Trimethyladipinsäure, Azelainsäure, Decandisäure, Dodecandisäure, Brassylsäure. Beispiele für carbocyclische Verbindungen sind: 1,2-Cyclohexandicarbonsäure (Hexahydrophthalsäure), 1,2-Cyclohexandicarbonsäureanhydrid (Hexahydrophthalsäureanhydrid), Cyclohexan-1,4-dicarbonsäure, Cyclohex-4-en-1,2-dicarbonsäure, Cyclohexen-1,2-dicarbonsäureanhydrid, 4-Methylcyclohexan-1,2-dicarbonsäure, 4-Methylcyclohexan-1,2-dicarbonsäureanhydrid, 4-Methylcyclohex-4-en-1,2-dicarbonsäure, 4-Methylcyclohex-4-en-1,2-dicarbonsäureanhydrid.

Beispiele geeigneter aromatischer Dicarbonsäuren bzw. deren Anhydride sind: Phthalsäure, Phthalsäureanhydrid, Isophthalsäure, Therephthalsäure, oder Naphthalindicarbonsäuren und deren Anhydride.

Beispiele geeigneter aromatischer Tricarbonsäuren (bzw. Anhydride) sind Trimellitsäure, Trimellitsäureanhydrid oder Trimesinsäure; Ein Beispiel einer geeigneten aromatischen Tetracarbonsäure bzw. ihres Anhydrids sind Pyromellitsäure und Pyromellitsäureanhydrid.

Besonders bevorzugt wird im erfindungsgemäßen Verfahren Phthalsäureanhydrid als Carbonsäurekomponente eingesetzt.

Im erfindungsgemäßen Verfahren werden bevorzugt verzweigte oder lineare aliphatische Alkohole mit 4 bis 13 C-Atomen, insbesondere 9 bis 13 C-Atomen, eingesetzt. Die Alkohole sind einwertig und können sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind beispielsweise n-Butanol, Isobutanol, n-Octanol(1), n-Octanol(2), 2-Ethylhexanol, Nonanole, Decylalkohole oder Tridecanoie hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Ein bevorzugtes Beispiel eines derartigen Alkoholgemisches ist ein C₉/C₁₁-Alkoholgemisch.

Bevorzugte Einsatzalkohole sind Gemische isomerer Octanole, Nonanole oder Tridecanole, wobei die letzteren aus den entsprechenden Butenoligomeren, insbesondere Oligomeren von linearen Butenen, durch Hydroformylierung und anschließender Hydrierung gewonnen werden können. Die Herstellung der Butenoligomeren kann im Prinzip nach drei Verfahren durchgeführt werden. Die sauer katalysierte Oligomerisierung, bei der technisch z. B. Zeolithe oder Phosphorsäure auf Trägem eingesetzt werden, liefert die verzweigtesten Oligomere. Bei Einsatz von linearen Butenen entsteht beispielsweise eine C₈-Fraktion, die im Wesentlichen aus Dimethylhexenen besteht (WO 92/13818). Ein ebenfalls weltweit ausgeübtes Verfahren ist die Oligomerisierung mit löslichen Ni-Komplexen, bekannt als DIMERSOL-Verfahren (B. Comils, W. A. Herrmann, Applied Homogenous Catalysis with Organometallic Compounds, Seite 261-263, Verlag Chemie 1996). Weiterhin wird die Oligomerisierung an Nickel-FestbettKatalysatoren ausgeübt, wie beispielsweise der OCTOL-Process (Hydrocarbon Process., Int. Ed. (1986) 65 (2. Sect. 1), Seite 31-33) oder das Verfahren gemäß der WO 95/14647 oder WO 01/36356.

Ganz besonders bevorzugte Einsatzstoffe für die erfindungsgemäße Veresterung sind Gemische isomerer Nonanole oder Gemische isomerer Tridecanole, die durch Oligomerisierung linearer Butene zu C₈-Olefinen und C₁₂-Olefinen nach dem Octol-Prozess oder gemäß der WO 95/14647, mit anschließender Hydroformylierung und Hydrierung hergestellt werden.

Weiterhin eignen sich Alkylenglykolmonoether, insbesondere Ethylenglykolmonoether, wie Ethylenglykolmonomethylether, Ethylenglykolmonoethylether und Ethylenglykölmonobuthylether; und Polyalkylenglykolmonoether insbesondere Polyethylenglykolmonoether, wie Polyethylenglykolmonomethylether.

Besonders bevorzugte Alkohohole sind 2-Ethylhexanol, 2-Propylheptanol, Isononanol-Isomerengemische, Decanol-Isomerengemische und C₈/C₁₁-Alkoholgemische.

Die erfindungsgemäße Veresterung wird in Gegenwart eines Veresterungskatalysators durchgeführt.

Geeigneterweise ist der Veresterungskatalysator unter Alkoholaten, Carboxylaten und Chelatverbindungen von Titan, Zirkonium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetrasec-butyltitanat, Tetraoctyltitanat, Tetra-(2-ethylhexyl)-titanat; Dialkyltitanate ((RO)₂TiO₂. worin R z.B. für iso-Propyl, n-Butyl, iso-Butyl steht), wie Isopropyl-n-butyltitanat; Titan-Acetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Din-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon-Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Aluminiumtris(ethylacetylacetonat). Insbesondere werden Isopropyl-n-butyltitanat, Tetra(isopropyl)orthotitanat oder Tetra(butyl)orthotitanat eingesetzt.

Die Katalysatorkonzentration hängt von der Art des Katalysators ab. Bei den bevorzugt eingesetzten Titanverbindungen beträgt diese 0,005 bis 1,0 Gew.-% bezogen auf das Reaktionsgemisch, insbesondere 0,01 bis 0,3 Gew.-%.

Bei diskontinuierlicher Verfahrensdurchführung können die Edukte und der Katalysator gleichzeitig oder nacheinander in den Reaktor eingefüllt werden. Der Katalysator kann in reiner Form oder als Lösung, bevorzugt gelöst in einem der Einsatzstoffe, zu Beginn oder erst nach Erreichen der Reaktionstemperatur eingebracht werden. Carbonsäureanhydride reagieren häufig mit Alkoholen bereits autokatalytisch, d. h. unkatalysiert zu den entsprechenden Estercarbonsäuren (Halbestem), beispielsweise Phthalsäureanhydrid zum Phthalsäuremonoester. Daher ist ein Katalysator häufig erst nach dem ersten Reaktionsschritt erforderlich.

Bei kontinuierlicher Verfahrensdurchführung führt man Ströme der Edukte und des Katalysators in den Reaktor bzw. bei Verwendung einer Reaktorkaskade in den ersten Reaktor der Kaskade ein. Die Verweilzeit im Reaktor bzw. den einzelnen Reaktoren wird dabei durch das Volumen der Reaktoren und den Mengenstrom der Edukte bestimmt.

Der umzusetzende Alkohol, der als Schleppmittel dient, kann im stöchiometrischen Überschuss, bevorzugt 30 bis 200 %, besonders bevorzugt 50 bis 100 % der stöchiometrisch notwendigen Menge eingesetzt werden.

Die Reaktionstemperaturen liegen zwischen 160 °C und 270 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Olefinbildung oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck und bei höher siedenden Alkoholen bei vermindertem Druck durchgeführt werden. Beispielsweise wird bei der Umsetzung von Phthalsäureanhydrid mit einem Gemisch isomerer Nonanole in einem Temperaturbereich von 170 °C bis 250 °C im Druckbereich von 200 mbar bis 3 bar gearbeitet.

Alle Reaktoren einer Kaskade können bei gleicher Temperatur betrieben werden. Im Allgemeinen ist es aber bevorzugt, die Temperatur vom ersten zum letzten Reaktor einer Kaskade stetig zu erhöhen, wobei ein Reaktor bei gleicher oder höherer Temperatur betrieben wird, als der (bezogen auf die Fließrichtung des Reaktionsgemisches) stromaufwärts gelegene Reaktor. Zweckmäßigerweise können alle Reaktoren bei im Wesentlichen gleichem Druck betrieben werden, insbesondere etwa Umgebungsdruck.

Nach Beendigung der Reaktion enthält das Reaktionsgemisch, das im Wesentlichen aus dem gewünschten Ester und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird der überschüssige Alkohol entfernt, die saueren Verbindungen neutralisiert, der Katalysator zerstört und die dabei entstandenen festen Nebenprodukte abgetrennt. Dabei wird der größte Teil des nicht umgesetzten Alkohols bei Normaldruck oder im Vakuum abdestilliert. Die letzten Spuren des Alkohols können z. B. durch Wasserdampfdestillation, insbesondere im Temperaturbereich von 120 bis 225 °C unter Vakuum, entfernt werden. Die Abtrennung des Alkohols kann als erster oder als letzter Aufarbeitungsschritt erfolgen.

Die Neutralisation der saueren Stoffe, wie Carbonsäuren, Estercarbonsäuren oder gegebenenfalls der saueren Katalysatoren, erfolgt durch Zugabe von Basen, z. B. von Alkali- und/oder Erdalkalimetallcarbonaten, -hydrogencarbonaten oder -hydroxiden. Das Neutralisationsmittel kann in fester Form oder bevorzugt als Lösung, insbesondere als wässrige Lösung eingesetzt werden. Hier wird häufig Natronlauge einer Konzentration von 1 bis 30 Gew.-%, bevorzugt von 20 bis 30 Gew.-% verwendet. Das Neutralisationsmittel wird in einer Menge zugesetzt, die dem Einfachen bis dem Vierfachen, insbesondere dem Einfachen bis Zweifachen der stöchiometrisch notwendigen Menge, die durch Titration bestimmt wird, entspricht.

Die so hergestellten Ester aus mehrbasischen Carbonsäuren, wie beispielsweise Phthalsäure, Adipinsäure, Sebacinsäure, Maleinsäure, und aus Alkoholen, finden weite Anwendung in Lackharzen, als Bestandteile von Anstrichmitteln und insbesondere als Weichmacher für Kunststoffe. Geeignete Weichmacher für PVC sind Dioctylphthalate, Diisononylphthalate, Diisodecylphthalate und Dipropylheptylphthalate.

Die Erfindung wird durch die beigefügte Zeichnung und die folgenden Beispiele näher erläutert.

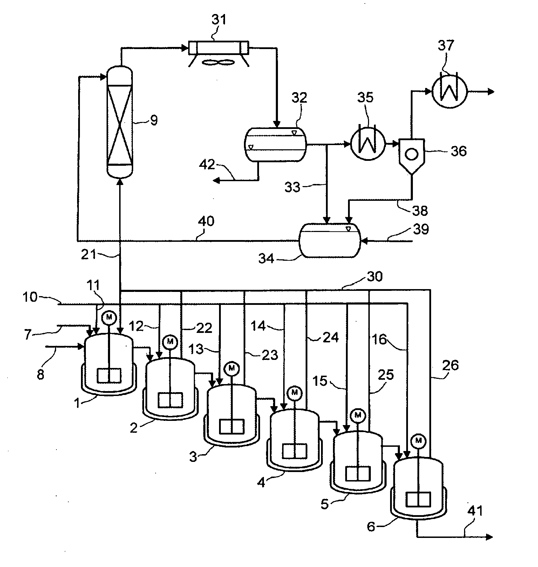

Fig. 1 zeigt eine zur Durchführung des erfindungsgemäßen Verfahrens geeignete Anlage. Die Anlage umfasst eine Kaskade aus sechs Rührkesseln 1, 2, 3, 4, 5 und 6, wobei der Ablauf des ersten Kessels dem zweiten Kessel, der Ablauf des zweiten Kessels dem dritten Kessel usw. zugeführt wird. Über die Alkohol-Sammelleitung 10 wird über die Zuleitungen 11, 12, 13, 14, 15 und 16 Alkohol in die Rührkessel 1, 2, 3, 4, 5 und 6 dosiert. Dem ersten Kessel 1 wird über die Leitung 8 Veresterungskatalysator zugesetzt. Über die Leitung 7 wird eine Säurekomponente, beispielsweise Phthalsäureanhydrd (PSA), in den ersten Kessel 1 gespeist.

Der Dampfraum des ersten Kessels 1 kommuniziert über die Leitung 21 mit der Rückalkohol-Kolonne 9, wobei die aus dem ersten Kessel 1 aufsteigenden Brüden über die Leitung 21 abgezogen werden und Rücklauf aus der Rückalkohol-Kolonne 9 ebenfalls über die Leitung 21 wieder dem ersten Kessel 1 zugeführt wird. Die Brüdenabzüge 22, 23, 24, 25, 26 aus dem zweiten bis sechsten Kessel 2, 3, 4, 5, 6 werden über die Brüdensammelleitung 30 zusammengefasst und führen über die Leitung 21 ebenfalls zur Rückalkohol-Kolonne 9.

Die vereinten Brüden werden einem Kondensator 31, z. B. einem luftgekühlten Kondensator, zugeführt. Der aus dem Kondensator 31 austretende gemischtphasige Strom wird im Phasenscheider 32 getrennt. Die untere, wässrige Phase wird über die Leitung 42 abgezogen. Die obere, organische Phase wird über die Leitung 33 teilweise dem Rückalkohol-Sammelgefäß 34 zugeleitet. Ein anderer Teil der organischen Phase aus dem Phasenscheider 32 wird im optionalen Wärmetauscher 35 erwärmt und in den Entspannungsbehälter 36 entspannt. Aufgrund der Entspannung trennt sich die organische Phase in eine Dampffraktion, in der die Leichtsieder angereichert sind, und eine Alkohol-angereicherte flüssige Fraktion. Die Dampffraktion kann im Nachkühler 37 kondensiert werden und einer Verwertung zugeführt werden. Die flüssige Fraktion wird über die Leitung 38 dem Rückalkohol-Sammelgefäß 34 zugeleitet. Über die Leitung 39 kann Alkohol, der bei der Aufarbeitung vom rohen Estergemisch abgetrennt wird, in das Rückalkohol-Sammelgefäß 34 geleitet und so ebenfalls der Wiederverwendung zugeführt werden. Über die Leitung 40 wird der Alkohol aus dem Rückalkohol-Sammelgefäß 34 am Kopf oder in den oberen Bereich der Rückalkohol-Kolonne 9 eingespeist, wo er den aufsteigenden Brüden entgegengeführt wird, und gelangt über die Leitung 21 zurück in den ersten Kessel 1.

### BEISPIELE

### Beispiel 1: Herstellung von Diisononylphthalat

Zur kontinuierlichen Herstellung von 2000 g/h Diisononylphthalat (DINP) verwendete man eine Kaskade von 4 Rührkesseln. In jeden Reaktionskessel wurde Isononanol zudosiert, insgesamt 1380 g/h Isononanol. In den ersten Reaktionskessel wurden 0,05 Gew.-% Isopropyl-n-butyltitanat, bezogen auf das Reaktionsgemisch, dosiert. Außerdem wurden 708 g/h Phthalsäureanhydrid (PSA) in den ersten Reaktionskessel dosiert. Über eine Rückalkohol-Kolonne am ersten Reaktor wurden etwa 1330 g/h Isononanolgemisch-Kreislaufrückstrom als Rücklauf auf die Rückalkohol-Kolonne gegeben.

Die Brüden aus dem ersten Reaktor wurden über die Rückalkohol-Kolonne abgezogen, deren Rücklauf in den ersten Reaktor zurückgeleitet wurde. Der Brüdenabzug aus dem zweiten bis vierten Reaktor erfolgte ebenfalls über die Rückalkohol-Kolonne.

Die Brüden aus der Veresterung wurden in einem Wasserkühler kondensiert und das Kondensat auf eine Temperatur von 70 °C gekühlt. In einem Phasenscheider wurden die organische und wässrige Phase bei Normaldruck getrennt. Das Wasser wurde ausgeschleust; ein Teil der organischen Phase (300 g/h; etwa 95 % Isononanol, 4 % Isononen) wurde über einen Alkoholsammelbehälter direkt in die Veresterung zurückgeführt.

149 g/h der organischen Phase wurden über einen Vorwärmer auf 100 °C erwärmt und in einen einstufigen Flash geleitet, der bei 100 mbar betrieben wurde. Die Dampfphase aus diesem Flash wurde in einem Nachkühler kondensiert (etwa 48 % Wasser, 23 % Isononanol, 29 % Isononen) und aus dem Verfahren ausgeschleust (7,3 g/h, davon 1,7 g/h Isononanol). Die flüssige, an Leichtsiedern (Isononen) abgereicherte Phase aus dem Flash (141,7 g/h, 97 % Isononanol) wurde in den Alkoholsammelbehälter geleitet und von dort in die Veresterung zurückgeführt.

Damit entstand ein Alkoholverlust von 0,85 g pro kg DINP (entsprechend 0,12 Mol-%-Punkte an Ausbeute).

### Vergleichsbeispiel 1

Die kontinuierliche Herstellung von DINP erfolgte analog zum Beispiel 1, wobei jedoch die im Phasenscheider abgezogene organische Phase ohne Nachbehandlung in die Veresterung zurückgeführt wurde. Um eine Anreicherung von Isononen im Isononanol-Kreislaufrückstrom zu verhindern, musste ein Teil der organischen Phase aus dem Verfahren kontinuierlich ausgeschleust werden.

Isononen-Gehalte von mehr als 5 Gew.-% im Isononanol-Kreislaufrückstrom können zu merklichen Beeinträchtigungen der Produktqualität führen. Um den Isononen-Gehalt im Isononanol-Kreislaufrückstrom auf maximal 5 Gew.-% zu begrenzen, mussten bei der Herstellung von 2000 g/h DINP 160 g/h organische Phase ausgeschleust werden, entsprechend einem Alkoholverlust von 76 g pro kg DINP (9,93 Mol-%-Punkte an Ausbeute).

### Beispiel 2: Herstellung von Dipropylheptylphthalat (mit einstufigem Flash zur Leichtsieder-Ausschleusung)

Zur kontinuierlichen Produktion von 1280 g/h Dipropylheptylphthalat aus PSA und 2-Propylheptanol (2-PH) in Gegenwart von Isopropyl-n-butyltitanat als Katalysator verwendete man eine Kaskade von 4 Rührkesseln. Die Brüden aus der Veresterung wurden kondensiert, und das Kondensat wird auf eine Temperatur von 85 °C gekühlt. In einem Phasenscheider wurden organische und wässrige Phase bei Normaldruck getrennt. Das Wasser wurde ausgeschleust.

Ein Teil der organischen Phase wurde über einen Vorwärmer auf 120 °C erwärmt und in einen Entspannungsbehälter entspannt, der auf 80 mbar gehalten wurde. Die bei der Entspannung gebildete Dampfphase wurde kondensiert (0,1 % Wasser, 27,8 % 2-PH, 72,1 % Decen) und aus dem Verfahren ausgeschleust (7,3 g/h, davon 2 g/h 2-PH). Die flüssige, an Leichtsiedern abgereicherte Phase aus der Entspannung wurde in einenen Alkoholsammelbehälter geleitet und von dort in die Veresterung zurückgeführt.

Damit entstand ein Alkoholverlust von 1,6 g pro kg Dipropylheptylphthalat (entsprechend 0,23 Mol-%-Punkte an Ausbeute).

### Beispiel 3: Herstellung von Dipropylheptylphthalat (mit Kolonne zur Leichtsieder-Ausschleusung)

Die Herstellung von 1280 g/h Dipropylheptylphthalat aus PSA und 2-Propylheptanol erfolgte analog zum Beispiel 2. Jedoch wurde ein Teil der organischen Phase über einen Vorwärmer auf 130 °C erwärmt und in eine Kolonne geleitet, die bei 80 mbar betrieben wurde. Die Dampfphase aus dieser Kolonne wurde kondensiert (12,7 g/h) und in einem Phasenscheider in eine organische und wässrige Phase getrennt. Die wässrige Phase (2,1 g/h, 99,9 Gew.-% Wasser, 0,1 Gew.-% Decen) wurde verworfen. Etwa die Hälfte der organischen Phase wurde als Rücklauf auf die Kolonne geleitet, der andere Teil wurde aus dem Verfahren ausgeschleust (5,2 g/h, reines Decen).

Das Sumpfprodukt der Kolonne wurde in den Alkoholsammelbehälter geleitet und von dort in die Veresterung zurückgeführt. (127,1 g/h, 86 % 2-PH, 14 % Decen). Damit wurde der Alkoholverlust über die Leichtsieder-Ausschleusung vollständig vermieden.

## Patentansprüche

1. Verfahren zur Herstellung von Carbonsäureestern durch Umsetzung einer Carbonsäure oder eines Carbonsäureanhydrids oder eines Gemisches davon mit einem Alkohol in Gegenwart eines Veresterungskatalysators, der unter Alkoholaten, Carboxylaten und Chelatverbindungen von Titan, Zirkonium, Zinn, Aluminium und Zink ausgewählt ist, in einem aus einem oder mehreren Reaktoren bestehenden Reaktionssystem, wobei man Reaktionswasser als Alkohol-Wasser-Azeotrop mit dem Brüden abdestilliert, den Brüden zumindest teilweise kondensiert, das Kondensat in eine wässrige Phase und eine organische Phase trennt und die organische Phase zumindest teilweise in das Reaktionssystem zurückführt, **dadurch gekennzeichnet, dass** man aus der zurückzuführenden organischen Phase niedriger als der Alkohol siedende Komponenten zumindest teilweise entfernt, wobei die niedriger als der Alkohol siedenden Komponenten Olefine umfassen, die sich durch Wasserabspaltung vom eingesetzten Alkohol ableiten.

2. Verfahren nach Anspruch 1, wobei man den Brüden unvollständig kondensiert, leichter als der Alkohol siedende Komponenten mit dem nicht kondensierten Brüden ausschleust, das Kondensat in eine wässrige Phase und eine organische Phase trennt und die organische Phase zumindest teilweise in das Reaktionssystem zurückführt.

3. Verfahren nach Anspruch 1, wobei man den Brüden zumindest teilweise kondensiert, das Kondensat in eine wässrige Phase und eine organische Phase trennt, zumindest einen Teil der organischen Phase behandelt, indem man niedriger als der Alkohol siedende Komponenten abdampft und/oder abdestilliert, und die so behandelte organische Phase zumindest teilweise in das Reaktionssystem zurückführt.

4. Verfahren nach Anspruch 3, wobei man einen Teil der organischen Phase unverändert in das Reaktionssystem zurückführt und einen anderen Teil der organischen Phase behandelt, indem man niedriger als der Alkohol siedende Komponenten abdampft und/oder abdestilliert, und die so behandelte organische Phase zumindest teilweise in das Reaktionssystem zurückführt.

5. Verfahren nach Anspruch 3 oder 4, wobei man zumindest einen Teil der organischen Phase in einen Entspannungsbehälter entspannt, wobei niedriger als der Alkohol siedende Komponenten zumindest teilweise verdampfen, und die unverdampfte flüssige Phase zumindest teilweise in das Reaktionssystem zurückführt.

6. Verfahren nach Anspruch 5, wobei man die organische Phase vor der Entspannung durch indirekten Wärmetausch erwärmt.

7. Verfahren nach einem der vorhergehenden Ansprüche, wobei die organische Phase über eine Kolonne in das Reaktionssystem zurückführt, in der man der rückgeführten organischen Phase zumindest einen Teil des Brüden entgegenführt.

8. Verfahren nach einem der vorhergehenden Ansprüche, wobei das Reaktionssystem eine Kaskade von mehreren Reaktoren umfasst.

9. Verfahren nach Anspruch 8, wobei man die organische Phase ausschließlich oder überwiegend in den ersten Reaktor der Kaskade zurückführt.

10. Verfahren nach einem der vorhergehenden Ansprüche, wobei die Carbonsäure unter aliphatischen Monocarbonsäuren mit wenigsten 5 Kohlenstoffatomen, aliphatischen C₄-C₁₀-Dicarbonsäuren, aromatischen Monocarbonsäuren, aromatischen Dicarbonsäuren, aromatischen Tricarbonsäuren, aromatischen Tetracarbonsäuren und Anhydriden davon ausgewählt ist.

11. Verfahren nach einem der vorhergehenden Ansprüche, wobei der Alkohol unter C₄-C₁₃-Alkoholen, Alkylenglykolmonoethern, Polyalkylenglykolmonoethern und Gemischen davon ausgewählt ist.

## Claims

1. A process for preparing carboxylic esters by reacting a carboxylic acid or a carboxylic anhydride or a mixture thereof with an alcohol in the presence of an esterification catalyst selected from among alkoxides, carboxylates and chelate compounds of titanium, zirconium, tin, aluminum and zinc, in a reaction system comprising one or more reactors, with water of reaction being distilled off as alcohol-water azeotrope with the vapor, the vapor being at least partly condensed, the condensate being separated into an aqueous phase and an organic phase and at least part of the organic phase being recirculated to the reaction system, wherein components having boiling points lower than that of the alcohol are at least partly removed from the organic phase to be recirculated, where the components having boiling points lower than that of the alcohol comprise olefins which are derived by elimination of water from the alcohol used.

2. The process according to claim 1, wherein the vapor is incompletely condensed, components having boiling points lower than that of the alcohol are discharged with the uncondensed vapor, the condensate is separated into an aqueous phase and an organic phase and the organic phase is at least partly recirculated to the reaction system.

3. The process according to claim 1, wherein the vapor is at least partly condensed, the condensate is separated into an aqueous phase and an organic phase, at least part of the organic phase is treated by evaporating and/or distilling off components having boiling points lower than that of the alcohol and at least part of the organic phase which had been treated in this way is recirculated to the reaction system.

4. The process according to claim 3, wherein part of the organic phase is recirculated unchanged to the reaction system and another part of the organic phase is treated by evaporating and/or distilling off components having boiling points lower than that of the alcohol and at least part of the organic phase which has been treated in this way is recirculated to the reaction system.

5. The process according to claim 3 or 4, wherein at least part of the organic phase is depressurized into a depressurization vessel, resulting in at least part of the components having boiling points lower than that of the alcohol evaporating, and the unvaporized liquid phase is at least partly recirculated to the reaction system.

6. The process according to claim 5, wherein the organic phase is heated by indirect heat exchange before the depressurization.

7. The process according to any of the preceding claims, wherein the organic phase is recirculated to the reaction system via a column in which the recirculated organic phase is conveyed in countercurrent to at least part of the vapor.

8. The process according to any of the preceding claims, wherein the reaction system comprises a cascade of a plurality of reactors.

9. The process according to claim 8, wherein the organic phase is recirculated exclusively or predominantly into the first reactor of the cascade.

10. The process according to any of the preceding claims, wherein the carboxylic acid is selected from among aliphatic monocarboxylic acids having at least 5 carbon atoms, aliphatic C₄-C₁₀-dicarboxylic acids, aromatic monocarboxylic acids, aromatic dicarboxylic acids, aromatic tricarboxylic acids, aromatic tetracarboxylic acids and anhydrides thereof.

11. The process according to any of the preceding claims, wherein the alcohol is selected from among C₄-C₁₃-alcohols, alkylene glycol monoethers, polyalkylene glycol monoethers and mixtures thereof.

## Revendications

1. Procédé pour la production d'esters d'acides carboxyliques par mise en réaction d'un acide carboxylique ou d'un anhydride d'acide carboxylique ou d'un mélange de ceux-ci en présence d'un catalyseur d'estérification qui est choisi parmi des alcoolates, carboxylates et composés chélate de titane, zirconium, étain, aluminium et zinc, dans un système réactionnel consistant en un ou plusieurs réacteurs, dans lequel on élimine par distillation avec la vapeur l'eau de réaction sous forme d'azéotrope alcool-eau, on condense au moins partiellement la vapeur, on fractionne le condensat en une phase aqueuse et une phase organique et on recycle au moins en partie la phase organique dans le système réactionnel, **caractérisé en ce qu'**on sépare au moins en partie de la phase organique à recycler les composants ayant un plus bas point d'ébullition que l'alcool, les composants ayant un plus bas point d'ébullition que l'alcool comprenant des oléfines qui dérivent par élimination d'eau de l'alcool utilisé.

2. Procédé selon la revendication 1, dans lequel on condense incomplètement la vapeur, on évacue avec la vapeur non condensée les composants ayant un plus bas point d'ébullition que l'alcool, on fractionne le condensat en une phase aqueuse et une phase organique et on recycle au moins en partie la phase organique dans le système réactionnel.

3. Procédé selon la revendication 1, dans lequel on condense au moins partiellement la vapeur, on fractionne le condensat en une phase aqueuse et une phase organique, on traite au moins une partie de la phase organique en éliminant par évaporation et/ou distillation les composants ayant un plus bas point d'ébullition que l'alcool, et on recycle au moins en partie la phase organique ainsi traitée dans le système réactionnel.

4. Procédé selon la revendication 3, dans lequel on renvoie inchangée une partie de la phase organique dans le système réactionnel et on traite une autre partie de la phase organique en éliminant par évaporation et/ou distillation les composants ayant un plus bas point d'ébullition que l'alcool, et on recycle au moins en partie la phase organique ainsi traitée dans le système réactionnel.

5. Procédé selon la revendication 3 ou 4, dans lequel on détend au moins une partie de la phase organique dans un récipient de détente, dans lequel les composants ayant un plus bas point d'ébullition que l'alcool sont au moins en partie évaporés, et on recycle au moins en partie dans le système réactionnel la phase liquide non évaporée.

6. Procédé selon la revendication 5, dans lequel avant la détente on chauffe la phase organique par échange thermique indirect.

7. Procédé selon l'une quelconque des revendications précédentes, dans lequel on renvoie la phase alcoolique dans le système réactionnel via une colonne dans laquelle on fait passer la phase alcoolique recyclée à contre-courant d'au moins une partie de la vapeur.

8. Procédé selon l'une quelconque des revendications précédentes, dans lequel le système réactionnel comprend une cascade de plusieurs réacteurs.

9. Procédé selon la revendication 8, dans lequel on renvoie la phase organique exclusivement ou principalement dans le premier réacteur de la cascade.

10. Procédé selon l'une quelconque des revendications précédentes, dans lequel l'acide carboxylique est choisi parmi des acides monocarboxyliques aliphatiques ayant au moins 5 atomes de carbone, des acides dicarboxyliques aliphatiques en C₄-C₁₀, des acides monocarboxyliques aromatiques, des acides dicarboxyliques aromatiques, des acides tricarboxyliques aromatiques, des acides tétracarboxyliques aromatiques et des anhydrides de ceux-ci.

11. Procédé selon l'une quelconque des revendications précédentes, dans lequel l'alcool est choisi parmi des alcools en C₄-C₁₃, des monoéthers d'alkylèneglycols, des monoéthers de polyalkylène-glycols et des mélanges de ceux-ci.
